# EUROPEAN PATENT APPLICATION

(11) **EP 1 700 585 A2**
(43) Date of publication of application: **13.09.2006**
(21) Application number: 06003666.2
(22) Date of filing: 23.02.2006
(51) Int. Cl.: A61F 13/15

(54) **Method of making absorbent core structures with undulations**

(30) Priority: 11.03.2005 US 906912
(71) Applicant: NORDSON CORPORATION, Westlake, Ohio 44145-1119 (US)
(72) Inventor: Bentley, Rachelle, Cumming,Georgia 30040 (US); Crane, Patrick L., Dawsonville,Georgia 30534 (US); Bernal, Stephen D., Cincinnati, Ohio 45240 (US); Davis, James H., Amelia, Ohio 45102 (US); Malakouti, Nezam, Loveland, Ohio 45140 (US)
(74) Representative: Eisenführ, Speiser & Partner

(57) **Abstract**

A method of making an absorbent core structure includes meltspinning at least one layer of fibrous material. At least one valley is formed separating at least two peaks in substantially parallel rows in the layer of fibrous material. A first portion of the first layer of fibrous material is folded over a second portion of the first layer of fibrous material. A least part of the first layer of fibrous material is densified.

## Description

### FIELD OF THE INVENTION

The present invention relates to absorbent core structures for disposable absorbent articles. More specifically, the present invention relates to absorbent core structures constructed of fibrous materials.

### BACKGROUND OF THE INVENTION

Disposable absorbent articles having absorbent core structures are well known in the art. Furthermore, it is well known that such absorbent core structures have at least three functional regions, namely, an acquisition region, a distribution region, and a storage region. While such regions are known, the design of absorbent core structures having said regions is limited by current methods of manufacture and current material selections.

One such conventional absorbent core structure includes the use of cellulosic materials. While the use of cellulosic materials provide satisfactory acquisition and distribution, often cellulosic core structures suffer from having poor wet integrity (i.e., has poor structural integrity when wet). In an effort to improve the wet integrity of such cellulosic core structures, the incorporation of expensive binders is often used. Another known problem when using cellulosic materials is the presence of knots and fines which are unsatisfactorily shaped fibers that negatively impact the core properties (e.g., efficacy, cost).

Another such conventional absorbent core structure includes the use of synthetic meltblown fibers. While the use of synthetic meltblown fibers provides satisfactory wet integrity, the resulting core structure is often limited in design. For example, synthetic meltblown fibers are generally small in diameter (e.g., 2 - 9 microns); thus, the resulting core structure would generally have poor acquisition properties. Further, these smaller fibers tend to be weak thus not permitting the creation of post-hydrated void areas. Additionally, synthetic meltblown core structures often require the use of expensive binders.

It is also well known that conventional absorbent core structures for use in disposable absorbent articles may be made of discrete, multiple layers of materials. Further, it is well known that said layers may consist of different types of materials. For example, a conventional absorbent article may be made of: (a) a top layer which serves as an acquisition region for more immediate absorption of exudate from the wearer, (b) an intermediate layer which serves as a distribution region for the intended transportation of exudate within the absorbent core structure (e.g., move exudate longitudinally or laterally for greater utilization of diaper) and (c) a bottom layer which serves as a storage region for more long-term storage of exudate.

What is needed is an absorbent core structure made of fibrous material in which properties of the acquisition region, distribution region, and storage region can be easily varied in the vertical and/or horizontal direction.

### SUMMARY OF THE INVENTION

An absorbent core structure having at least one acquisition region, at least one distribution region, and at least one storage region. The acquisition region being constructed from a fibrous material. The acquisition region having a relatively low density from about 0.018 g/cc to about 0.20 g/cc. The distribution region being constructed from said fibrous material. The distribution region being consolidated to have a relatively medium density from about 0.024 g/cc to about 0.45 g/cc. The distribution region being in fluid communication with said acquisition region. The storage region being constructed from said fibrous material. The storage region being consolidated to have a relatively high density from about 0.030 g/cc to about 0.50 g/cc. The storage region being in fluid communication with said distribution region. A portion of the fibrous material being formed into at least one peak and at least one valley and then subsequently folded in order to form said absorbent core structure. The fibrous material may be selected from the group consisting of polypropylene, polyethylene, polyester, polyvinyl alcohol, polyvinyl acetate, starch, cellulose acetate, polybutane, rayon, urethane, Kraton™, polylactic acid, cotton, Lyocell™, biogradeable polymers, any other material which is suitable for forming a fiber, and combinations thereof. The absorbent core structure may also include a superabsorbent material, such as a superabsorbent polymer (SAP) and/or other materials with superabsorbent properties. The SAP may be deposited onto at least one of said valley. The SAP may be deposited onto at least one of said peak. The SAP may be deposited onto at least one of said valley and onto at least one of said peak. The SAP may be deposited onto alternating valleys. The SAP may be deposited onto alternating peaks. A first row of said peaks may align substantially vertically with a second row of said peaks. A first row of said peaks may align substantially vertically with a first row of said valleys. The fibrous material may include a linear portion substantially free of peaks and valleys. The linear portion may be folded and positioned between at least two layers of peaks and valleys. SAP may be deposited onto the linear portion.

An absorbent core structure having at least one acquisition region, at least one distribution region, and at least one storage region. The acquisition region being constructed from a first fibrous material. The acquisition region having a relatively low density from about 0.018 g/cc to about 0.20 g/cc. The distribution region being constructed from a second fibrous material. The distribution region being consolidated to have a relatively medium density from about 0.024 g/cc to about 0.45 g/cc. The distribution region being in fluid communication with the acquisition region. The distribution region having at least one peak and at least one valley. The storage region being constructed from a third fibrous material. The storage region being consolidated to have a relatively high density from about 0.030 g/cc to about 0.50 g/cc. The storage region being in fluid communication with said distribution region. The storage region having at least one peak and at least one valley. The first fibrous material, second fibrous material and third fibrous material being laid on top of each other in order to form said absorbent core structure. The fibrous materials may be selected from the group consisting of polypropylene, polyethylene, polyester, polyvinyl alcohol, polyvinyl acetate, starch, cellulose acetate, polybutane, rayon, urethane, Kraton™, polylactic acid, cotton, Lyocell™, biogradeable polymers, any other material which is suitable for forming a fiber, and combinations thereof. The absorbent core structure may also include a superabsorbent material, such as a superabsorbent polymer (SAP) and/or other materials having superabsorbent properties. The SAP may be deposited onto at least one of said valley. The SAP may be deposited onto at least one of said peak. The SAP may be deposited onto at least one of said valley and onto at least one of said peak. The SAP may be deposited onto alternating valleys. The SAP may be deposited onto alternating peaks. A row of said peaks within said distribution region may align substantially vertically with a row of said peaks within said storage region. A row of said peaks within said distribution region may align substantially vertically with a row of said valleys within said storage region.

The invention further contemplates various methods of making an absorbent core structure, such as for use in a disposable hygienic product. In general, the methods can involve meltspinning at least one layer of fibrous material, forming at least two peaks and at least one valley in the layer of fibrous material, and densifying at least a portion of that layer. In the various embodiments, a superabsorbent material may be utilized for fluid storage purposes. The superabsorbent material may be formed from polymers and/or other materials. The meltspinning process may, for example, involve meltblowing and/or spunbonding processes that deposit fibers on a moving collector such as a conveying element formed from wire.

In one particular illustrative embodiment, a first layer of fibrous material is formed having at least two peaks and at least one valley separating the peaks. A first portion of the first layer of fibrous material is folded over a second portion of the first layer of fibrous material, and at least part of the first layer of fibrous material is densified. The height of the layer at each peak may be several times the height at each valley as measured from an opposite surface of the layer of fibrous material. For example, a 9:1 ratio may be useful for certain applications. However, a higher or lower ratio may be desirable as well. Currently, a minimum ratio of about 2:1 is preferred for use in the present invention. The width and shapes of the peaks and valleys may also be varied as desired.

Various additional features may also optionally be used in connection with the embodiment described above, or other embodiments of the invention. For example, forming the first layer of fibrous material can desirably involve forming multiple, alternating peaks and valleys along a first surface of the first layer of fibrous material. Densifying at least part of the first layer can further involve densifying at least a portion of the multiple peaks. Folding the first portion of the first layer of fibrous material over the second portion of the first layer of fibrous material may further comprise aligning respective pairs of the peaks in opposing relation, or aligning the peaks with opposed valleys, or vice versa, or combinations of these two options, depending on the desired density characteristics. The peaks and/or other areas of the first layer of fibrous material may be compressed or otherwise densified uniformly across the entire layer, or in a selected portion or portions depending on the desired fluid acquisition, distribution and/or storage properties imparted or assisted by such compression.

In another aspect, a third portion of the fibrous layer may be folded between the first and second portions. In any of these embodiments having multiple layers or multiple layer portions positioned adjacent to each other, a superabsorbent material may be deposited onto one or more layers or layer portions uniformly or at spaced apart locations, depending on the needs of the particular article to be manufactured. The superabsorbent material may also or alternatively be dispersed within the fibers that make up one or more of the fibrous layers or layer portions.

In embodiments in which first and second discrete layers are used, with or without folding of one or both layers, the fibers that make up the first and second layers may be formed of the same material or from materials having different properties.

An illustrative embodiment that involves the use of discrete layers of fibrous material to form an absorbent core structure includes forming a first layer of a first fibrous material having at least one valley separating at least two peaks. A second layer of a second fibrous material is placed against the peaks and the valley. The method further includes densifying the fibrous material forming the peaks of the first layer and an area of the second layer placed against the peaks. The second layer may be generally flat, or may include one or more peaks and valleys. This embodiment, like the others of this invention, may also have additional layers depending on the needs of the application, and may include other features such as those described above, used alone or in any desired combinations.

Various additional features, advantages and objectives of the invention will become more readily apparent to those of ordinary skill in the art upon review of the following detailed description of the preferred embodiments taken in conjunction with the accompanying drawings.

### BRIEF DESCRIPTION OF THE DRAWINGS

FIG. 1a shows an exemplary portion of a fibrous material having peaks and valleys;

FIG. 1b shows the fibrous material of FIG. 1 wherein a prior peak is shown having been substantially collapsed such that a shorter, denser region results;

FIG. 2a shows an exemplary fibrous material being folded;

FIG. 2b shows the absorbent core structure of FIG. 2a being densified such that the resulting caliper is decreased and many of the densities are increased;

FIG. 2c shows a close-up view of the encircled area of FIG. 2b whereupon the regions of varying densities may be further appreciated;

FIG. 3a shows an exemplary fibrous material being folded;

FIG. 3b shows the absorbent core structure of FIG. 3a being densified such that the resulting caliper is decreased and many of the densities are increased;

FIG. 3c shows a close-up view of the encircled area of FIG. 3b whereupon the regions of varying densities may be further appreciated;

FIG. 4a shows an exemplary fibrous material being folded;

FIG. 4b shows the absorbent core structure of FIG. 4a being densified such that the resulting caliper is decreased and many of the densities are increased;

FIG. 4c shows a close-up view of the encircled area of FIG. 4b whereupon the regions of varying densities may be further appreciated;

FIG. 5a shows an exemplary fibrous material being folded;

FIG. 5b shows the absorbent core structure of FIG. 5a being densified such that the resulting caliper is decreased and many of the densities are increased;

FIG. 5c shows a close-up view of the encircled area of FIG. 5b whereupon the regions of varying densities may be further appreciated;

FIG. 6a shows an exemplary fibrous material being folded;

FIG. 6b shows the absorbent core structure of FIG. 6a being densified such that the resulting caliper is decreased and many of the densities are increased;

FIG. 6c shows a close-up view of the encircled area of FIG. 6b whereupon the regions of varying densities may be further appreciated;

FIG. 7a shows an exemplary fibrous material being folded;

FIG. 7b shows the absorbent core structure of FIG. 7a being densified such that the resulting caliper is decreased and many of the densities are increased;

FIG. 7c shows a close-up view of the encircled area of FIG. 7b whereupon the regions of varying densities may be further appreciated;

FIG. 8a shows an exemplary fibrous material being folded;

FIG. 8b shows the absorbent core structure of FIG. 8a being densified such that the resulting caliper is decreased and many of the densities are increased;

FIG. 8c shows a close-up view of the encircled area of FIG. 8b;

FIG. 9a shows an exemplary fibrous material being folded;

FIG. 9b shows the absorbent core structure of FIG. 9a being densified such that the resulting caliper is decreased and many of the densities are increased;

FIG. 9c shows a close-up view of the encircled area of FIG. 9b whereupon the regions of varying densities may be further appreciated;

FIG. 10a shows an exemplary fibrous material being folded;

FIG. 10b shows the absorbent core structure of FIG. 10a being densified such that the resulting caliper is decreased and many of the densities are increased;

FIG. 10c shows a close-up view of the encircled area of FIG. 10b whereupon the regions of varying densities may be further appreciated;

FIG. 11a shows a first discrete mid-layer of fibrous material having peaks and valleys and a second discrete layer of fibrous material having a first undulating region and a second undulating region, each having peaks and valleys;

FIG. 11b shows said second undulating region being consolidated onto first discrete mid-layer such that their aligned peaks are further densified, while their aligned valleys still provide a void space;

FIG. 11c shows a close-up view of the encircled area of FIG. 11b whereupon the regions of varying densities may be further appreciated;

FIG. 12a shows an exemplary laid-down approach comprising a first layer of fibrous material having peaks and valleys, a second layer of fibrous material having peaks and valleys and a third layer of fibrous material being substantially planar;

FIG. 12b shows said third layer being consolidated onto said second layer such that said third layer substantially fills valleys;

FIG. 12c shows a close-up view of the encircled area of FIG. 12b whereupon the regions of varying densities may be further appreciated;

FIG. 13a shows a two-dimensional schematic view of an absorbent core having acquisition regions, distribution regions and storage regions being selectively placed throughout the core design;

FIG. 13b shows a three-dimensional schematic of FIG. 13a with fluid moving therein;

FIG. 13c shows a three-dimensional schematic of FIG. 13b with fluid moving further therein; and

FIG. 14 shows a three-dimensional schematic view of another absorbent core having acquisition regions, distribution regions and storage regions vary in their three-dimensional placement.

### DETAILED DESCRIPTION OF THE EMBODIMENTS

Various definitions of terms used herein are provided as follows:

The term "absorbent article" herein refers to devices which absorb and contain body exudates and, more specifically, refers to devices which are placed against or in proximity to the body of the wearer to absorb and contain the various exudates discharged from the body, such as: incontinence briefs, incontinence undergarments, absorbent inserts, diaper holders and liners, feminine hygiene garments and the like. The absorbent article may have an absorbent core having a garment surface and a body surface; a liquid permeable topsheet positioned adjacent the body surface of the absorbent core; and a liquid impermeable backsheet positioned adjacent the garment surface of the absorbent core.

The term "disposable" is used herein to describe absorbent articles which generally are not intended to be laundered or otherwise restored or reused as absorbent articles (i.e., they are intended to be discarded after a single use and, preferably, to be recycled, composted or otherwise discarded in an environmentally compatible manner).

The term "diaper" herein refers to an absorbent article generally worn by infants and incontinent persons about the lower torso.

The term "pant", as used herein, refers to disposable garments having a waist opening and leg openings designed for infant or adult wearers. A pant may be placed in position on the wearer by inserting the wearer's legs into the leg openings and sliding the pant into position about the wearer's lower torso. A pant may be preformed by any suitable technique including, but not limited to, joining together portions of the article using refastenable and/or non-refastenable bonds (e.g., seam, weld, adhesive, cohesive bond, fastener, etc.). A pant may be preformed anywhere along the circumference of the article (e.g., side fastened, front waist fastened). While the term "pant" is used herein, pants are also commonly referred to as "closed diapers", "prefastened diapers", "pull-on diapers", "training pants" and "diaper-pants". Suitable pants are disclosed in U.S. Patent No. 5,246,433, issued to Hasse, et al. on September 21, 1993; U.S. Patent No. 5,569,234, issued to Buell et al. on October 29, 1996; U.S. Patent No. 6,120,487, issued to Ashton on September 19, 2000; U.S. Patent No. 6,120,489, issued to Johnson et al. on September 19, 2000; U.S. Patent No. 4,940,464, issued to Van Gompel et al. on July 10, 1990; U.S. Patent No. 5,092,861, issued to Nomura et al. on March 3, 1992; U.S. Patent Application Serial No. 10/171,249, entitled "Highly Flexible And Low Deformation Fastening Device", filed on June 13, 2002; U.S. Patent No. 5,897,545, issued to Kline et al. on April 27, 1999; U.S. Patent No. 5,957,908, issued to Kline et al on September 28, 1999.

The term "machine direction (MD)" or "longitudinal" herein refers to a direction running parallel to the maximum linear dimension of the article and/or fastening material and includes directions within ±45° of the longitudinal direction.

The term "cross direction (CD)", "lateral" or "transverse" herein refers to a direction which is orthogonal to the longitudinal direction.

The term "joined" encompasses configurations whereby an element is directly secured to another element by affixing the element directly to the other element, and configurations whereby an element is indirectly secured to another element by affixing the element to intermediate member(s) which in turn are affixed to the other element.

As used herein the term "spunbond fibers" refers to small diameter fibers of substantially molecularly oriented polymeric material. Spunbond fibers are generally formed by extruding molten thermoplastic material as filaments from a plurality of fine, usually circular capillaries of a spinneret with the diameter of the extruded filaments then being rapidly reduced by an attenuation process. Spunbond fibers are generally not tacky when they are deposited onto a collecting surface and are generally continuous.

As used herein the term "spunbond material" refers to material made from spunbond fibers.

As used herein the term "meltblown fibers" means fibers of polymeric material which are generally formed by extruding a molten thermoplastic material through a plurality of fine, usually circular, die capillaries as molten threads or filaments into converging high velocity, usually hot, gas (e.g. air) streams which attenuate the filaments of molten thermoplastic material to reduce their diameter. Thereafter, the meltblown fibers can be carried by the high velocity gas stream and are deposited on a collecting surface to form a web of randomly dispersed meltblown fibers. Meltblown fibers may be continuous or discontinuous, are generally smaller than 10 microns in average diameter, and are generally tacky when deposited onto a collecting surface.

As used herein the term "polymer" generally includes but is not limited to, homopolymers, copolymers, such as for example, block, graft, random and alternating copolymers, terpolymers, etc. and blends and modifications thereof. Furthermore, unless otherwise specifically limited, the term "polymer" includes all possible spatial configurations of the molecule. These configurations include, but are not limited to isotactic, syndiotactic and random symmetries.

As used herein, "ultrasonic bonding" means a process performed, for example, by passing the fabric between a sonic horn and anvil roll.

As used herein the term "acquisition layer" or "acquisition region" means a fibrous material having a relatively low density from about 0.018 g/cc to about 0.20 g/cc and a relatively high caliper from about 0.41 mm to about 5.23 mm.

As used herein the term "distribution layer" or "distribution region" means a fibrous material having a relatively medium density from about 0.024 g/cc to about 0.45 g/cc and a relatively medium caliper from about 0.39 mm to about 4.54 mm.

As used herein the terms "storage layer" or "storage region" mean any region that contains SAP. Further, the terms mean a fibrous material having a relatively high density from about 0.03 g/cc to about 0.50 g/cc and a relatively low caliper 0.15 mm to about 3.96 mm.

As used herein the term "small diameter" describes any fiber with a diameter of less than or equal to 10 microns.

As used herein the term "large diameter' describes any fiber with a diameter of greater than 10 microns.

As used herein the term "superabsorbent" refers to a material that can absorb at least about 10 times its weight in fluid.

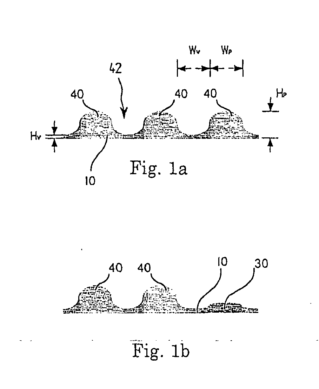

FIG. 1 a shows an exemplary portion of a fibrous material 10 having peaks 40 and valleys 42. Peaks 40 have a general height of about 9 mm to about 35 mm (shown as Hp; preferably about 27 mm) and a general width of about 2.5 mm to about 25 mm (shown as Wp; preferably about 12 mm). Valleys 42 have a general height of about 1 mm to about 17.4 mm (shown as Hᵥ; preferably about 3 mm) and a general width of about 2.5 mm to about 25 mm (shown as Wᵥ; preferably about 12 mm). Peaks have a general basis weight of about 99% to about 51 % as compared to the valleys' basis weight of about 1 % to about 49%. For example, assuming an average basis weight of 100 gsm, the peaks may have a basis weight of about 90% (or about 180 gsm with a corresponding height of about 9 mm) and the valleys may have a basis weight of about 10% (or about 20 gsm with a corresponding height of about 1 mm). The fibers of fibrous material 10 may be made of a variety of suitable materials including, but not limited to, polypropylene, polyethylene, polyester, polyvinyl alcohol, polyvinyl acetate, starch, cellulose acetate, polybutane, rayon, polyurethane, Kraton™, polylactic acid, cotton, Lyocell™, biogradeable polymers, any other material which is suitable for forming a fiber, and combinations thereof. The fibrous fibers of the present invention may have a diameter from about 10 micron to about 600 microns, unlike conventional meltblown fibers which typically have a diameter from about 2 to about 9 microns. Having such a larger diameter allows for the creation of low density fibrous materials which provide the necessary void space for acquisition layers. Being able to modify the density is also necessary in order to provide distribution and storage areas. Such modification techniques include, but are not limited to, consolidation (e.g., nip rolls, vacuum while attenuating fibers in a manufacturing beam, etc.), calendering (e.g., nip rolls with heat), ultrasonic and through air bonding (as exampled in U.S. Patent No. 4,011,124.

FIG. 1 b shows the fibrous material 10 of FIG. 1 wherein a prior peak is shown having been substantially collapsed such that a shorter, denser region 30 results. The fundamental idea of a peak being collapsed into a shorter, denser region will be further illustrated in the following embodiments.

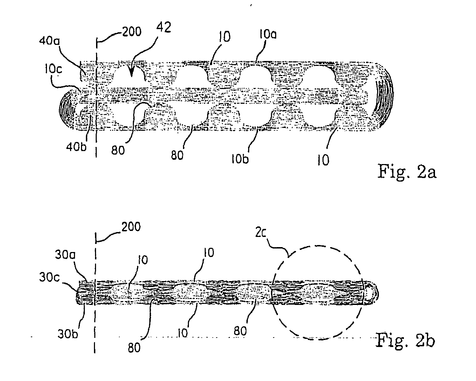

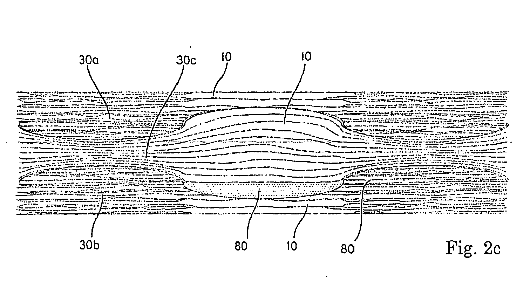

FIG. 2a shows an exemplary fibrous material 10 being folded. This particular exemplary embodiment is shown being tri-folded. Fibrous material 10 may comprise of regions having peaks 40 and valleys 42. Fibrous material 10 may also comprise regions without peaks 40 and valleys 42. For example, undulating regions 10a and 10b may have peaks 40 and valleys 42, while planar region 10c does not have peaks and valleys. Planar region 10c may be positioned between undulating regions 10a and 10b to create a multi-layer absorbent core structure. Planar region 10c may help to entrap SAP and also to maintain overall structural integrity by keeping the SAP in position so as not to create a shear line within the overall core structure. In this particular exemplary embodiment, the peaks 40a and 40b of undulating regions 10a and 10b, respectively, are substantially-vertically aligned as shown by line 200. Prior to the folding of fibrous material 10, super absorbent polymer 80 (hereinafter SAP) may be deposited in the valleys 42 and partially on the peaks of undulating region 10b. For example, assuming a deposition amount of 8.4 grams within an absorbent core structure having dimensions of 100 mm x 350 mm, the corresponding apparent bulk density may equal about 0.67 g/cc with a height of about 0.362 mm. FIG. 2b shows the absorbent core structure of FIG. 2a being densified such that the resulting caliper is decreased and many of the densities are increased. For example, areas where the peaks 40 were vertically aligned now have relatively high densities 30a, 30b, 30c because of the presence of more material as compared to the lesser amount of material in the valleys 42. As can also be seen, the void spaces of valleys 42 are now substantially filled such that the regions above SAP 80 have relatively low densities 10. This exemplary embodiment offers a particular advantage over the prior art in that the acquisition region and the distribution region are side by side. This allows longitudinal distribution of exudate as the void spaces in the acquisition region begin to fill. FIG. 2c shows a close-up view of the encircled area of FIG. 2b whereupon the regions of varying densities may be further appreciated.

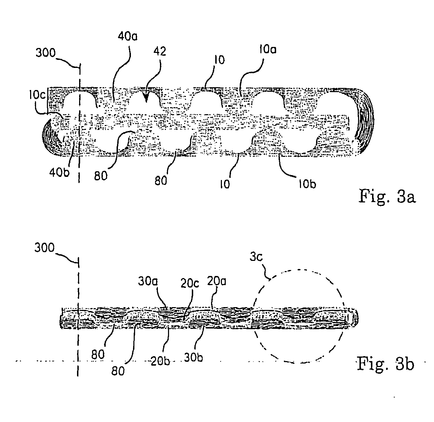

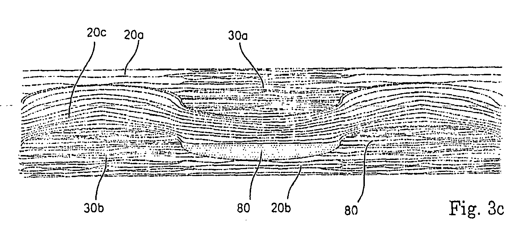

FIG. 3a shows an exemplary fibrous material 10 being folded. This particular exemplary embodiment is shown being tri-folded. Fibrous material 10 may comprise of regions having peaks 40 and valleys 42. Fibrous material 10 may also comprise regions without peaks 40 and valleys 42. For example, undulating regions 10a and 10b may have peaks 40 and valleys 42, while planar region 10c does not have peaks and valleys. Planar region 10c may be positioned between undulating regions 10a and 10b to create a multi-layer absorbent core structure. Planar region 10c may help to entrap SAP and also to maintain overall structural integrity by keeping the SAP in position so as not to create a shear line within the overall core structure. In this particular exemplary embodiment, the peaks 40a and 40b of undulating regions 10a and 10b, respectively, are not substantially-vertically aligned as shown by line 300. Prior to the folding of fibrous material 10, SAP 80 may be deposited in the valleys 42 and partially on the peaks of undulating region 10b. FIG. 3b shows the absorbent core structure of FIG. 3a being densified such that the resulting caliper is decreased and many of the densities are increased. For example, areas where the peaks and valleys were aligned now have relatively high densities 30a, 30b on one end and relatively medium densities 20a, 20b on the other end. As can also be seen, the void spaces of valleys 42 are now substantially filled such that the regions above SAP 80 have relatively medium densities 20c. FIG. 3c shows a close-up view of the encircled area of FIG. 3b whereupon the regions of varying densities may be further appreciated.

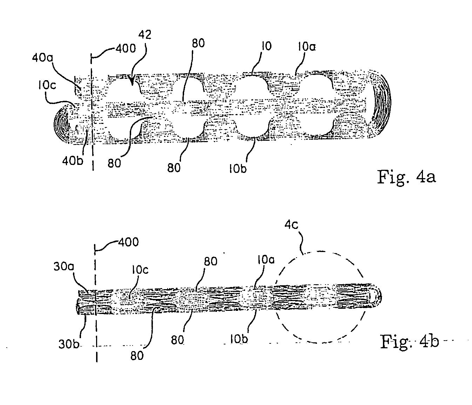

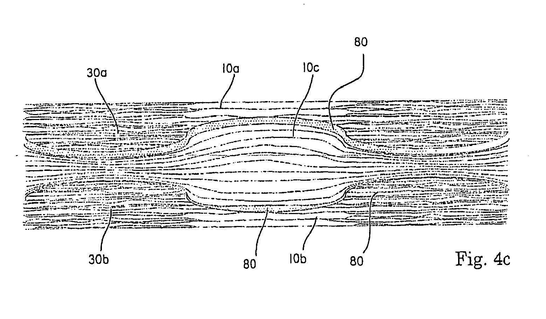

FIG. 4a shows an exemplary fibrous material 10 being folded. This particular exemplary embodiment is shown being tri-folded. Fibrous material 10 may comprise of regions having peaks 40 and valleys 42. Fibrous material 10 may also comprise regions without peaks 40 and valleys 42. For example, undulating regions 10a and 10b may have peaks 40 and valleys 42, while planar region 10c does not have peaks and valleys. Planar region 10c may be positioned between undulating regions 10a and 10b to create a multi-layer absorbent core structure. Planar region 10c may help to entrap SAP and also to maintain overall structural integrity by keeping the SAP in position so as not to create a shear line within the overall core structure. In this particular exemplary embodiment, the peaks 40a and 40b of undulating regions 10a and 10b, respectively, are substantially-vertically aligned as shown by line 400. Prior to the initial fold, SAP 80 may be deposited in the valleys and partially on the peaks. Additionally, prior to the final fold, SAP 80 may be deposited on the top side of planar region 10c. FIG. 4b shows the absorbent core structure of FIG. 4a being densified such that the resulting caliper is decreased and many of the densities are increased. For example, areas where the peaks were vertically aligned now have relatively high densities 30a, 30b. As can also be seen, the void spaces of valleys 42 are now substantially filled such that the regions above SAP 80 have relatively low densities 10c. As can also be appreciated, this exemplary embodiment provides two areas of SAP 80, one continuous layer of SAP above planar region 10c and another area consisting of discrete depositions of SAP in the valleys and peaks under the planar region 10c. FIG. 4c shows a close-up view of the encircled area of FIG. 4b whereupon the regions of varying densities may be further appreciated.

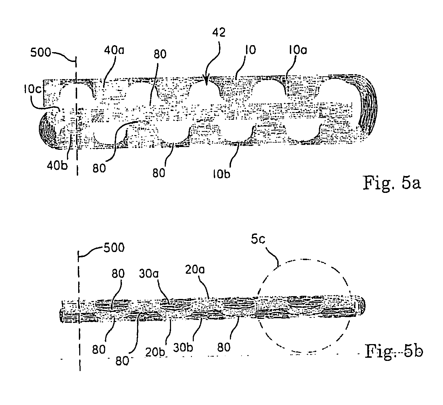

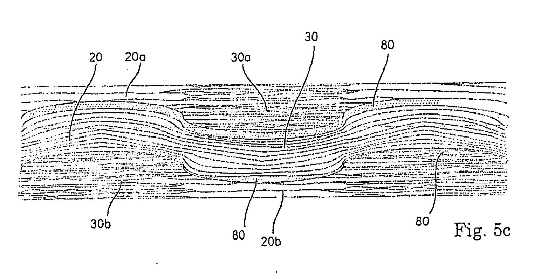

FIG. 5a shows an exemplary fibrous material 10 being folded. This particular exemplary embodiment is shown being tri-folded. Fibrous material 10 may comprise of regions having peaks 40 and valleys 42. Fibrous material 10 may also comprise regions without peaks 40 and valleys 42. For example, undulating regions 10a and 10b may have peaks 40 and valleys 42, while planar region 10c does not have peaks and valleys. Planar region 10c may be positioned between undulating regions 10a and 10b to create a multi-layer absorbent core structure. Planar region 10c may help to entrap SAP and also to maintain overall structural integrity by keeping the SAP in position so as not to create a shear line within the overall core structure. In this particular exemplary embodiment, the peaks 40a and 40b of undulating regions 10a and 10b, respectively, are not substantially-vertically aligned as shown by line 500. Prior to the initial fold, SAP 80 is deposited in the valleys and partially on the peaks. Additionally, prior to the final fold, SAP 80 is deposited on the top side of planar region 10c. FIG. 5b shows the absorbent core structure of FIG. 5a being densified such that the resulting caliper is decreased and many of the densities are increased. For example, areas where the peaks and valleys were aligned now have relatively high densities 30a, 30b on one end and relatively medium densities 20a, 20b on the other end. As can also be appreciated, this exemplary embodiment provides two areas of SAP 80, one continuous layer of SAP above planar region 10c and another area consisting of discrete depositions of SAP in the valleys and peaks under the planar region 10c. FIG. 5c shows a close-up view of the encircled area of FIG. 5b whereupon the regions of varying densities may be further appreciated.

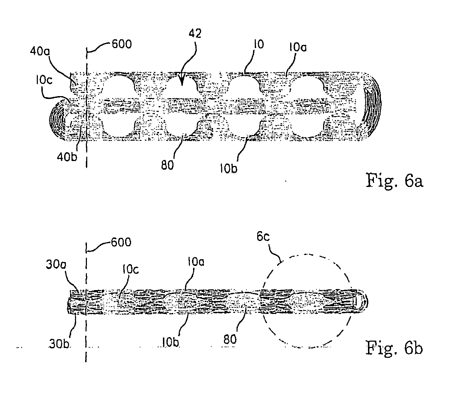

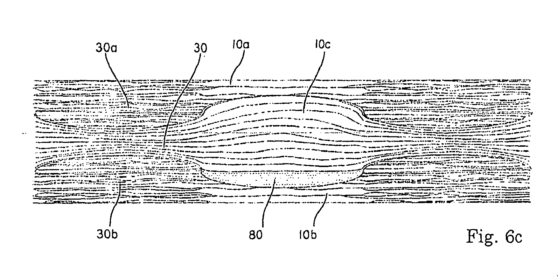

FIG. 6a shows an exemplary fibrous material 10 being folded. This particular exemplary embodiment is shown being tri-folded. Fibrous material 10 may comprise of regions having peaks 40 and valleys 42. Fibrous material 10 may also comprise regions without peaks 40 and valleys 42. For example, undulating regions 10a and 10b may have peaks 40 and valleys 42, while planar region 10c does not have peaks and valleys. Planar region 10c may be positioned between undulating regions 10a and 10b to create a multi-layer absorbent core structure. Planar region 10c may help to entrap SAP and also to maintain overall structural integrity by keeping the SAP in position so as not to create a shear line within the overall core structure. In this particular exemplary embodiment, the peaks 40a and 40b of undulating regions 10a and 10b, respectively, are substantially-vertically aligned as shown by line 600. Prior to the initial fold, SAP 80 is deposited in the valleys and not on the peaks. To achieve such AGM deposition, special care can be made to ensure insignificant deposition on the peaks or additional processes (e.g. blowing air to top of peaks) may be incorporated to remove any original deposition of SAP. FIG. 6b shows the absorbent core structure of FIG. 6a being densified such that the resulting caliper is decreased and many of the densities are increased. For example, areas where the peaks were vertically aligned now have relatively high densities 30a, 30b. As can also be seen, the void spaces of valleys 42 are now substantially filled such that the regions above SAP 80 have relatively low densities 10c. FIG. 6c shows a close-up view of the encircled area of FIG. 6b whereupon the regions of varying densities may be further appreciated.

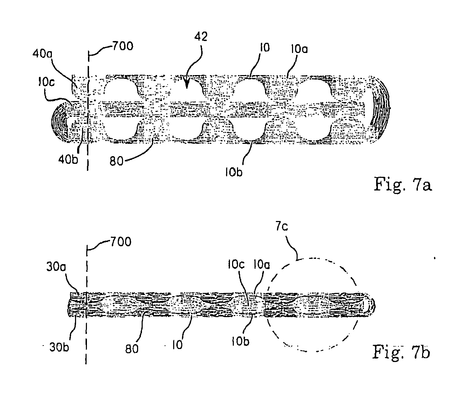

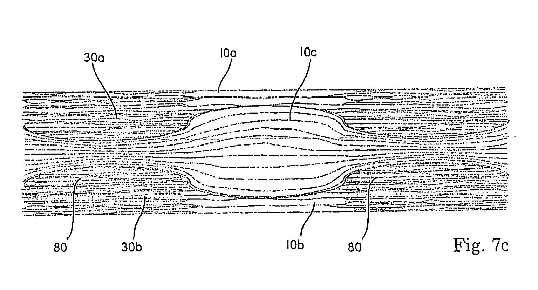

FIG. 7a shows an exemplary fibrous material 10 being folded. This particular exemplary embodiment is shown being tri-folded. Fibrous material 10 may comprise of regions having peaks 40 and valleys 42. Fibrous material 10 may also comprise regions without peaks 40 and valleys 42. For example, undulating regions 10a and 10b may have peaks 40 and valleys 42, while planar region 10c does not have peaks and valleys. Planar region 10c may be positioned between undulating regions 10a and 10b to create a multi-layer absorbent core structure. Planar region 10c may help to entrap SAP and also to maintain overall structural integrity by keeping the SAP in position so as not to create a shear line within the overall core structure. In this particular exemplary embodiment, the peaks 40a and 40b of undulating regions 10a and 10b, respectively, are substantially-vertically aligned as shown by line 700. Prior to the initial fold, SAP 80 is deposited on the peaks and not in the valleys. To achieve such SAP deposition, special care may be made to ensure insignificant deposition in the valleys and/or additional processes (e.g., blowing air within valleys) may be incorporated to remove any original deposition of SAP. FIG. 7b shows the absorbent core structure of FIG. 7a being densified such that the resulting caliper is decreased and many of the densities are increased. For example, areas where the peaks were vertically aligned now have relatively high densities 30a, 30b. As can also be seen, the void spaces of valleys 42 are now substantially filled such that the corresponding regions now have relatively low densities 10c. As can also be appreciated, the deposition of SAP 80 is vertically surrounded by relatively high densities 30a, 30b and is horizontally surrounded by relatively low densities 10c. FIG. 7c shows a close-up view of the encircled area of FIG. 7b whereupon the regions of varying densities may be further appreciated.

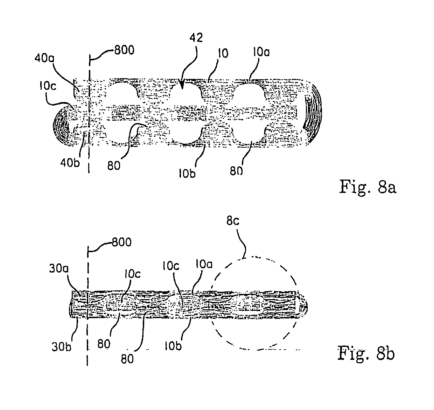

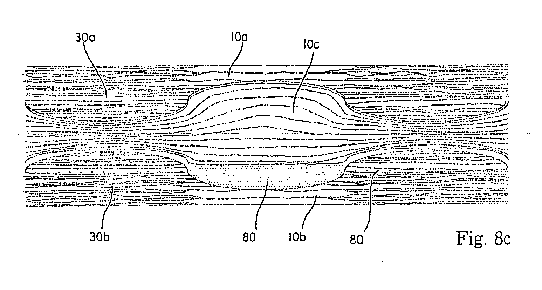

FIG. 8a shows an exemplary fibrous material 10 being folded. This particular exemplary embodiment is shown being tri-folded. Fibrous material 10 may comprise of regions having peaks 40 and valleys 42. Fibrous material 10 may also comprise regions without peaks 40 and valleys 42. For example, undulating regions 10a and 10b may have peaks 40 and valleys 42, while planar region 10c does not have peaks and valleys. Planar region 10c may be positioned between undulating regions 10a and 10b to create a multi-layer absorbent core structure. Planar region 10c may help to entrap SAP and also to maintain overall structural integrity by keeping the SAP in position so as not to create a shear line within the overall core structure. In this particular exemplary embodiment, the peaks 40a and 40b of undulating regions 10a and 10b, respectively, are substantially-vertically aligned as shown by line 800. Prior to the initial fold, SAP 80 may be deposited in alternating valleys and partially on the peaks. Since SAP tends to significantly swell in the presence of fluid, providing alternating valleys without SAP provides for later available acquisition regions for subsequent urine insults. To achieve such SAP deposition, special care may be made to assure such deposition and/or additional processes (e.g., blowing air within alternating valleys) may be incorporated to remove any original deposition of SAP. FIG. 8b shows the absorbent core structure of FIG. 8a being densified such that the resulting caliper is decreased and many of the densities are increased. For example, areas where the peaks were vertically aligned now have relatively high densities 30a, 30b. As can also be seen, the void spaces of valleys 42 are now substantially filled such that the regions within the valleys have relatively low densities 10c. FIG. 8c shows a close-up view of the encircled area of FIG. 8b whereupon the regions of varying densities may be further appreciated.

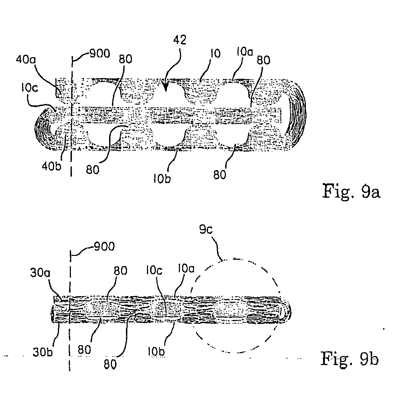

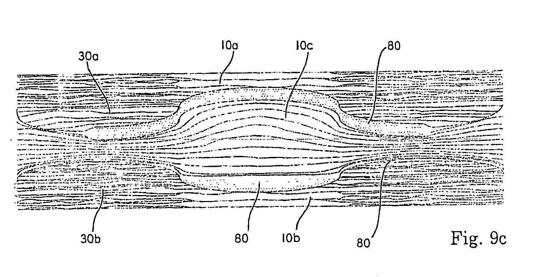

FIG. 9a shows an exemplary fibrous material 10 being folded. This particular exemplary embodiment is shown being tri-folded. Fibrous material 10 may comprise of regions having peaks 40 and valleys 42. Fibrous material 10 may also comprise regions without peaks 40 and valleys 42. For example, undulating regions 10a and 10b may have peaks 40 and valleys 42, while planar region 10c does not have peaks and valleys. Planar region 10c may be positioned between undulating regions 10a and 10b to create a multi-layer absorbent core structure. Planar region 10c may help to entrap SAP and also to maintain overall structural integrity by keeping the SAP in position so as not to create a shear line within the overall core structure. In this particular exemplary embodiment, the peaks 40a and 40b of undulating regions 10a and 10b, respectively, are substantially-vertically aligned as shown by line 900. Prior to the initial fold, SAP 80 may be deposited in alternating valleys and partially on the peaks. Since SAP tends to significantly swell in the presence of fluid, providing alternating valleys without SAP provides for later available acquisition regions for subsequent urine insults. To achieve such SAP deposition, special care may be made to assure such deposition and/or additional processes (e.g., blowing air within alternating valleys) may be incorporated to remove any original deposition of SAP. Additionally, prior to the final fold, SAP 80 may be deposited on the top side of planar region 10c in a discontinuous manner. This second deposition layer of SAP may or may not be substantially similar to the first deposition layer. For example, the upper layer of SAP may be slower acting in order to allow a first urine insult to be stored by the lower layer and then permit the upper layer to be available for subsequent urine insults. Furthermore, the upper layer of SAP may be cheaper than the lower layer, thus providing a cost savings without inferior efficacy. FIG. 9b shows the absorbent core structure of FIG. 9a being densified such that the resulting caliper is decreased and many of the densities are increased. For example, areas where the peaks were vertically aligned now have relatively high densities 30a, 30b. As can also be seen, the void spaces of valleys 42 are now substantially filled such that the regions above SAP 80 have relatively low densities 10c. As can also be appreciated, this exemplary embodiment provides two areas of SAP 80, a discontinuous layer of SAP above planar region 10c and another area consisting of discrete depositions of SAP in the valleys and peaks under the planar region 10c. FIG. 9c shows a close-up view of the encircled area of FIG. 9b whereupon the regions of varying densities may be further appreciated.

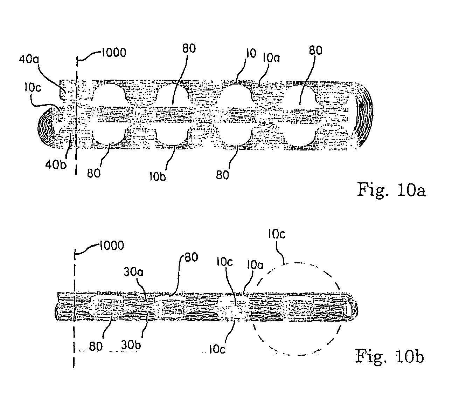

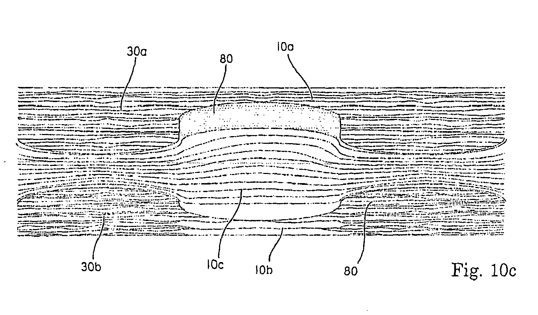

FIG. 10a shows an exemplary fibrous material 10 being folded. This particular exemplary embodiment is shown being tri-folded. Fibrous material 10 may comprise of regions having peaks 40 and valleys 42. Fibrous material 10 may also comprise regions without peaks 40 and valleys 42. For example, undulating regions 10a and 10b may have peaks 40 and valleys 42, while planar region 10c does not have peaks and valleys. Planar region 10c may be positioned between undulating regions 10a and 10b to create a multi-layer absorbent core structure. Planar region 10c may help to entrap SAP and also to maintain overall structural integrity by keeping the SAP in position so as not to create a shear line within the overall core structure. In this particular exemplary embodiment, the peaks 40a and 40b of undulating regions 10a and 10b, respectively, are substantially-vertically aligned as shown by line 1000. Prior to the initial fold, SAP 80 may be deposited in alternating valleys and not on the peaks. Since SAP tends to significantly swell in the presence of fluid, providing alternating valleys without SAP provides for later available acquisition regions for subsequent urine insults. To achieve such SAP deposition, special care may be made to assure such deposition and/or additional processes (e.g., blowing air within alternating valleys and along the peaks) may be incorporated to remove any original deposition of SAP. Additionally, prior to the final fold, SAP 80 may be deposited on the top side of planar region 10c in a discontinuous manner such that the SAP is located substantially in the valleys 42. This second deposition layer of SAP may or may not be substantially similar to the first deposition layer. For example, the upper layer of SAP may be slower acting in order to allow a first urine insult to be stored by the lower layer and then permit the upper layer to be available for subsequent urine insults. Furthermore, the upper layer of SAP may be cheaper than the lower layer, thus providing a cost savings without inferior efficacy. FIG. 10b shows the absorbent core structure of FIG. 10a being densified such that the resulting caliper is decreased and many of the densities are increased. For example, areas where the peaks were vertically aligned now have relatively high densities 30a, 30b. As can also be seen, the void spaces of valleys 42 are now substantially filled such that the regions within the valleys have relatively low densities 10c. As can also be appreciated, this exemplary embodiment provides two areas of SAP 80, a discontinuous layer of SAP above planar region 10c and another area consisting of discrete depositions of SAP in alternating valleys under the planar region 10c. FIG. 10c shows a close-up view of the encircled area of FIG. 10b whereupon the regions of varying densities may be further appreciated.

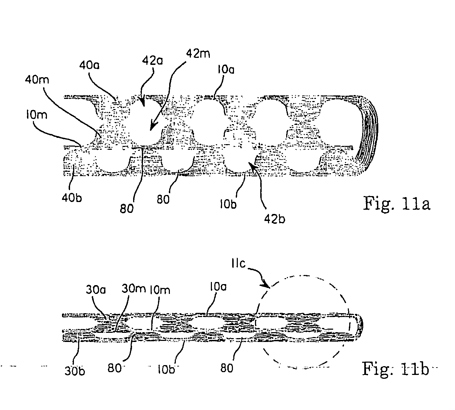

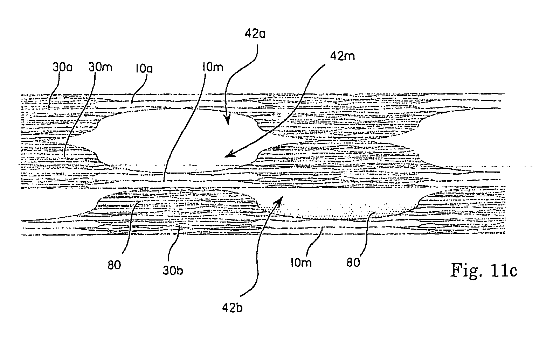

In an alternate approach to providing a substantially planar midportion which is folded, FIG. 11a shows a first discrete mid-layer of fibrous material 10m having peaks 40m and valleys 42m and a second discrete layer of fibrous material having a first undulating region 10a and a second undulating region 10b, each having peaks 40a, 40b and valleys 42a, 42b, respectively. The second layer being folded around the first layer. The second layer may help to entrap SAP and also to maintain overall structural integrity by keeping the SAP in position so as not to create a shear line within the overall core structure. In this particular embodiment, second undulating region 10b and first discrete mid-layer 10m may be positioned such that the valleys of one layer vertically aligns with the peaks of the other layer as shown by line 1100. Alternatively, one skilled in the art would appreciate that the peaks of each layer may be vertically aligned. Similar positioning possibilities exist between the first undulating region 10a and the first discrete mid-layer 10m. Prior to folding, SAP 80 may be deposited in some or all of valleys 42b and on some or all of the peaks 40b. While not shown, SAP 80 may be deposited in some or all of valleys 42m and on some or all of the peaks 40m. FIG. 11 b shows the second undulating region 10a being consolidated onto first discrete mid-layer 10m such that their aligned peaks are further densified, while their aligned valleys still provide a void space. Further, peaks 40b of second undulating region 10b may provide non-aligned structural support to the aligned peaks above, thus providing a second region of void spaces. FIG. 11c shows a close-up view of the encircled area of FIG. 11b whereupon the regions of varying densities may be further appreciated.

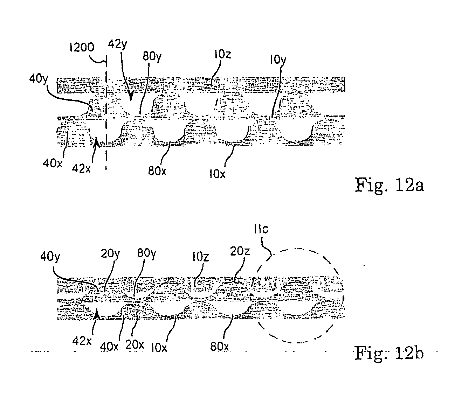

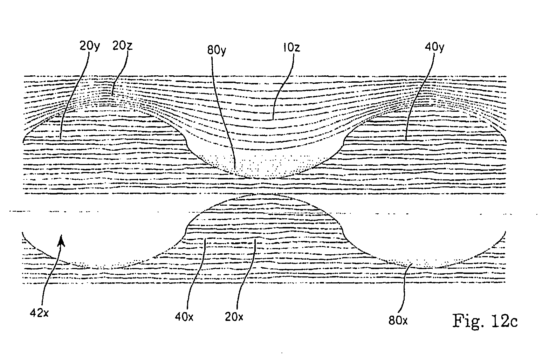

In an alternate approach to folding, FIG. 12a shows an exemplary laid-down approach comprising a first layer of fibrous material 10x having peaks 40x and valleys 42x, a second layer of fibrous material 10y having peaks 40y and valleys 42y and a third layer of fibrous material 10z being substantially planar. The second and third layers may help to entrap SAP and also to maintain overall structural integrity by keeping the SAP in position so as not to create a shear line within the overall core structure. In this particular embodiment, first layer of fibrous material 10x and second layer of fibrous material 10y may be positioned such that the valleys of one layer vertically aligns with the peaks of the other layer as shown by line 1200. Alternatively, one skilled in the art would appreciate that the peaks of each layer may be vertically aligned. Further, in this particular embodiment, SAP 80x may be deposited in some or all of valleys 42x and SAP 80y may be deposited in some or all of valleys 42y. Additionally, the third layer of fibrous material 10z may be positioned on top of or between the first and second layers. The upper deposition layer of SAP may or may not be substantially similar to the lower deposition layer. For example, the upper layer of SAP may be slower acting in order to allow a first urine insult to be stored by the lower layer and then permit the upper layer to be available for subsequent urine insults. Furthermore, the upper layer of SAP may be cheaper than the lower layer, thus providing a cost savings without inferior efficacy. FIG. 12b shows the third layer 10z being consolidated onto the second layer 10y such that the third layer 10z substantially fills valleys 42y. In this particular embodiment, the valleys 42x of first layer 10x remain substantially intact, while peaks 40x now have a relatively medium density. FIG. 12c shows a close-up view of the encircled area of FIG. 12b whereupon the regions of varying densities may be further appreciated.

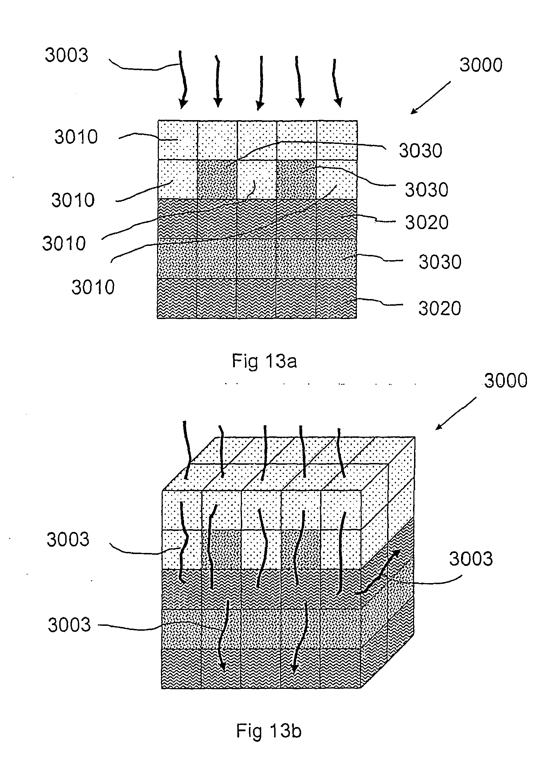

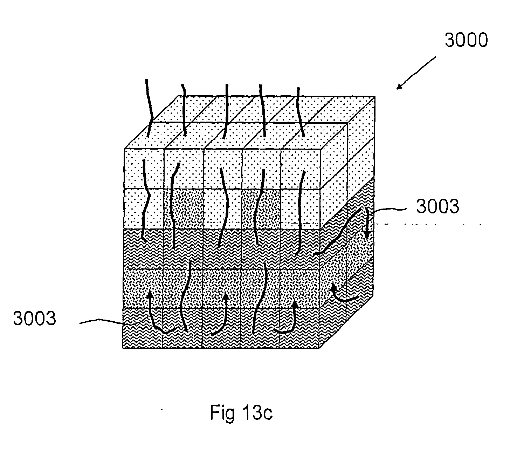

Referring now to FIG. 13a, a two-dimensional schematic is shown to depict one of the benefits of the present invention. More specifically, the novel aspects of the present invention provide for the creation of novel core structure designs. For instance, FIG. 13a shows a two-dimensional schematic view of an absorbent core 3000 having acquisition regions 3010, distribution regions 3020 and storage regions 3030 being selectively placed throughout the core design. Such a designs provides for novel fluid management.

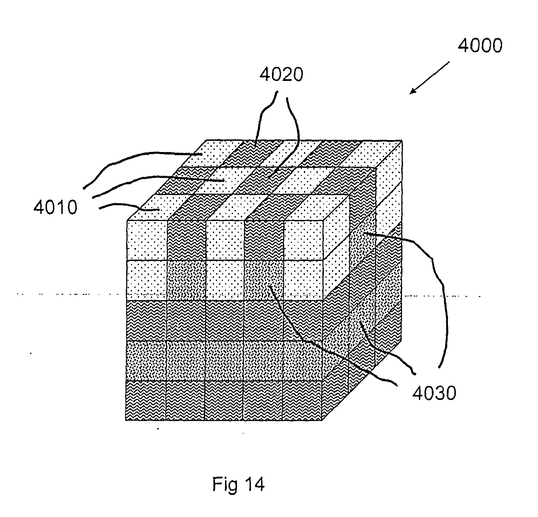

It is well known that conventional absorbent core structures for use in disposable absorbent articles may be made of multiple layers of materials. Further, it is well known that the layers may consist of different types of materials. For example, a conventional absorbent article may be made of: (a) a top layer which serves as an acquisition region for more immediate absorption of exudate from the wearer, (b) an intermediate layer which serves as a storage region for more long-term storage of exudate and (c) a bottom layer which serves as a distribution region for the intended transportation of exudate within the absorbent core structure (e.g., move exudate longitudinally or laterally for greater utilization of diaper). However, such conventional cores often do not permit inter-layer fluid communication. Not only does the present invention provide inter-layer fluid communication, but it provides three-dimensional fluid management as depicted in the series of FIGS. 13a - 13c, wherein the fluid 3003 is moved in accordance with the core design principles disclosed herein. Lastly, the core structure may be designed to have its regions (i.e., acquisition regions 4010, distribution regions 4020 and storage regions 4030) vary in their three-dimensional placement as depicted by absorbent core 4000 in FIG. 14.

All documents cited in the Detailed Description of the Invention are, in relevant part, incorporated herein by reference; the citation of any document is not to be construed as an admission that it is prior art with respect to the present invention.

Various methods and devices may be used to form the peaks 40 and valleys 42 in the one or more layers of fibrous material 10 to carry out the invention. These may include techniques that are integrated into a meltspinning process or techniques that are implemented after formation of the layer(s), or a combination of such processes. The preferred manner of forming peaks 40 and valleys 42 is one that is integrated into the meltspinning process. In this regard, for example, the apparatus and methods disclosed in U.S. Patent Application Serial No. 10/714,778, (the '778 application) filed on November 17, 2003, the disclosure of which is hereby incorporated by reference herein, may be used to achieve a striping effect in a layer of fibrous, spunbond material. The striping effect produces rows of higher density material in the form of peaks separated by rows of lower density material in the form of valleys. To achieve this, the distance of the attenuator or draw jet outlet of the spunbond apparatus is moved closer than normal to the fibrous material collector. For example, if this distance, referenced as "ACD" in the '778 application, is about 10" to produce a fibrous material layer of uniform density, then an ACD of about 5" may produce the desired striping or peaks 40 and valleys 42 in the fibrous material layer 10 for purposes of the present invention. It will be appreciated that other methods, including but not limited to those that involve contacting a layer of fibrous material with a shaping element after the layer is produced, may be used as well. Each row of the peaks 40 and valleys 42 may alternatively be continuous or discontinuous, depending on the needs of the application.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

For example, one skilled in the art would appreciate varying degrees of consolidation.

For example, one skilled in the art would appreciate that SAP deposition may be accomplished in a variety of suitable techniques including, but not limited to, registered deposition after peaks and valleys are formed or depositing SAP during peak/valley formation (e.g., add SAP into top of manufacturing beam attenuator wherein the SAP should follow the fibers around the diffusing members).

## Claims

1. A method of making an absorbent core structure, comprising:
meltspinning at least one layer of fibrous material,
forming at least one valley separating at least two peaks in substantially parallel rows in the layer of fibrous material,
folding a first portion of the first layer of fibrous material over a second portion of the first layer of fibrous material, and
densifying at least part of the first layer of fibrous material.

2. The method of claim 1, wherein the forming step further comprises:
forming multiple peaks alternating with multiple valleys along a first surface of the first layer of fibrous material.

3. The method of claim 2, wherein densifying at least part of the first layer of fibrous material further comprises:
densifying at least a portion of the multiple peaks.

4. The method of claim 2, wherein folding a first portion of the first layer of fibrous material over a second portion of the first layer of fibrous material further comprises:
aligning respective pairs of the peaks in opposing relation.

5. The method of claim 4, wherein densifying at least part of the first layer of fibrous material further comprises:
compressing the respective pairs of the peaks in opposing relation.

6. The method of claim 5, further comprising:
folding a third portion of the fibrous layer between the first and second portions.

7. The method of claim 2, wherein folding a first portion of the first layer of fibrous material over a second portion of the first layer of fibrous material further comprises:
aligning respective pairs of the peaks and valleys in opposing relation.

8. The method of claim 7, wherein densifying at least part of the first layer of fibrous material further comprises:
compressing the respective peaks in opposing relation to the valleys.

9. The method of claim 8, further comprising:
folding a third portion of the fibrous layer between the first and second portions.

10. The method of claim 2, further comprising:
depositing a superabsorbent material between the first and second portions of the first layer of fibrous material.

11. The method of claim 2, further comprising:
depositing a superabsorbent material into at least some of the valleys.

12. The method of claim 2, further comprising:
depositing a superabsorbent material onto at least some of the peaks.

13. The method of claim 2, further comprising:
dispersing a superabsorbent material within the first layer of fibrous material.

14. The method of claim 2, further comprising:
depositing first and second amounts of superabsorbent material in spaced apart relation between the first and second portions of the first layer of fibrous material.

15. The method of claim 2, further comprising:
positioning a second layer of fibrous material between the first and second portions of the first layer of fibrous material.

16. The method of claim 15, wherein fibers forming the first layer have different properties than fibers forming the second layer.

17. The method of claim 5, further comprising:
depositing a superabsorbent material onto at least one of the first and second layers.

18. A method of making an absorbent core structure from a layer of fibrous material, comprising:
meltspinning at least a first layer of fibrous material,
forming at least one valley separating at least two peaks in substantially parallel rows in the first layer of fibrous material,
placing a second layer of a second fibrous material against the peaks and the valley, and
densifying the fibrous material forming the peaks of the first layer and an area of the second layer placed against the peaks.

19. The method of claim 18, wherein the forming step further comprises:
forming multiple peaks alternating with multiple valleys along a first surface of the first layer of fibrous material.

20. The method of claim 19, wherein densifying the fibrous material further comprises:
densifying each peak and each corresponding area of the second layer placed against each peak.

21. The method of claim 20, further comprising:
depositing a superabsorbent material between the first and second layers.

22. The method of claim 18, wherein the second layer further comprises at least one peak and at least one valley.

23. The method of claim 22, further comprising:
positioning a third generally flat layer of fibrous material against at least one of the first and second layers of fibrous material.
